(19) Europäisches Patentamt
European Patent Office
Office européen des brevets

(11) EP 3 998 271 A2

(12) EUROPEAN PATENT APPLICATION

(43) Date of publication:
**18.05.2022 Bulletin 2022/20**

(21) Application number: **21204074.5**

(22) Date of filing: **21.10.2021**

(51) International Patent Classification (IPC):
***C07D 487/04*** $^{(2006.01)}$ ***H01L 51/00*** $^{(2006.01)}$

(52) Cooperative Patent Classification (CPC):
**C07D 487/04; H01L 51/0072**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.10.2020 PT 2020116848**

(71) Applicants:
- **UNIVERSIDADE NOVA DE LISBOA**
  **1099-085 Lisboa (PT)**
- **NOVA.ID.FCT - ASSOCIAÇÃO PARA A INOVAÇÃO E DESENVOLVIMENTO DA FCT**
  **2825-516 Monte Da Caparica (PT)**
- **Universidade de Évora**
  **7000-803 Évora (PT)**

(72) Inventors:
- **BURKE, ANTHONY JOSEPH**
  **ÉVORA (PT)**
- **SILVA MARQUES, CAROLINA**
  **BENEDITA (PT)**
- **CRISTOVAO ALMEIDA PRATES RAMALHO, JOÃO PAULO**
  **ÉVORA (PT)**
- **ALMEIDA FERNANDES COBRA BRANCO, LUIS ALEXANDRE**
  **SOBREDA (PT)**
- **NUNES GAGO, SANDRA MARIA**
  **FERNÃO FERRO (PT)**
- **DA SILVA CRUZ, HUGO GONÇALO**
  **CALDAS DA RAINHA (PT)**

(74) Representative: **Alves Moreira, Pedro**
**RCF Protecting Innovation, S.A.**
**Rua Dom Francisco Manuel de Melo, 15, 3°**
**1070-085 Lisbon (PT)**

# (54) PENTACYCLIC DIAMIDES WITH OPTOELECTRONIC APPLICATIONS, METHODS TO PRODUCE THEM AND USES THEREOF

(57) The present application discloses the novel pentacyclic diamides of formula (I) and their analogues of formula (II), their use as potential applications in optoelectronic devices. Both pentacyclic amide families of formula (I) and formula (II) are obtained via novel synthetic methods described herein.

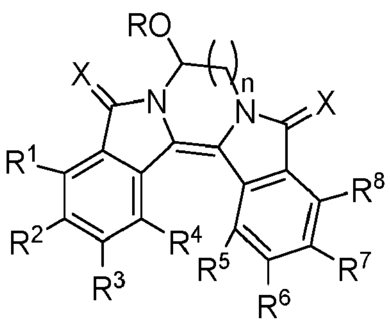

I

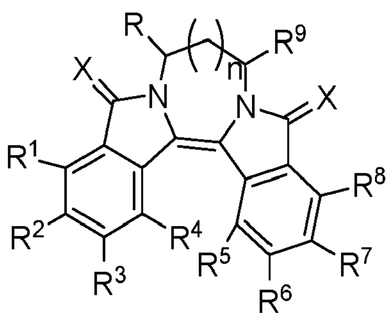

II

EP 3 998 271 A2

## Description

### Technical field

The present application describes novel pentacyclic diamides of formula (I) and their analogues of formula (II) and their use as components for optoelectronic devices, and methods to produce these compounds.

### Background art

At the current time the development of novel and more efficient optoelectronic devices has become a center of much focus in both chemistry and materials science, particularly with regard to novel energy harvesting and molecular imagining/detection systems. In this context, there has been an increased focus on novel organic electronics (Feron et *al.* 2018). Developing novel semi-conductor systems for light-harvesting purposes with clean energy production in mind is a very important goal and much attention is being devoted to this (Wang et *al.* 2018). At the same time, research into the development of novel chromophoric systems, generally using fluorescence probes, is also important, especially with regard to bio-imagining systems for biological and biomedical applications (Wysockia and Lavis, 2011). The design and application of fluorescent small molecules (fluorophores) as sensors/probes in biomedical applications have been one of the most exciting innovations in the life sciences area to date. Coumarins, xanthenes, like rhodamine and fluorescein, cyanines, as well as, boron-dipyrromethene (BODIPY) and Nile Red are most commonly used for this purpose, many of them being commercially available. The present application describes compounds of formula (**I**) and (**II**), their use as components for optoelectronic devices, and methods to produce the referred compounds.

### General description

It is the goal of this application to disclose the families of pentacyclic diamides with application as components for optoelectronic devices and methods to produce these compounds.

Thus, the present application describes the use of compounds of formula (**I**) and (**II**):

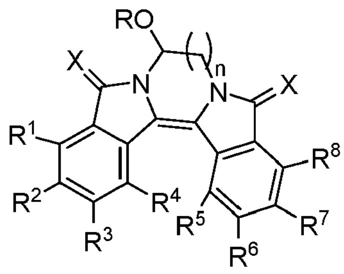

I

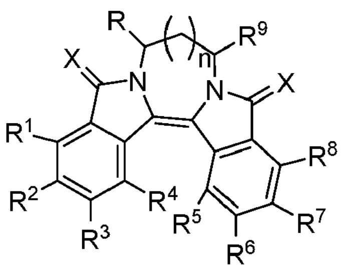

II

wherein, X is O, S or Se,

n = 1-4 for compounds of formula (**I**) and n = 0-3 for compounds of formula (**II**),
R to $R^9$ represent: H, alkyl, aryl, vinyl, allyl, alkoxyl, OH, CN, CHO, $CO_2H$, $CO_2R$ where R represents any alkyl or aryl group, halogen: F, Br, Cl.

In the case of $R^1$ to $R^8$ the adjacent pairs, including the pair R and $R^9$ (for example, $R^1$ and $R^2$, etc), can represent fused aromatic or cycloalkyl units.

These compounds can be chiral and exist in one enantiomeric form - either pure or enriched - as diastereomers - either enantiomerically pure or enriched - or as a racemic mixture.

The novel compounds of formula (**I**) are obtained from compounds of formula (**III**):

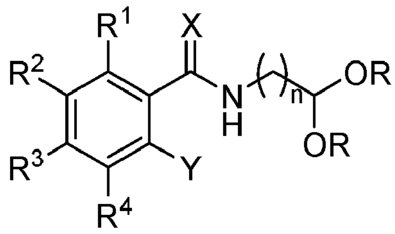

III

The compounds of formula (**I**) are obtained from compounds of formula (**III**), in which Y is a halogen and n = 1-3.
In one embodiment the halogen is, but not limited to, Br, Cl or I. Other options include triflate ($OSO_2CF_3$) and alkylsulfonates.
The compounds of formula (**II**) are obtained from compounds of formula (**IV**):

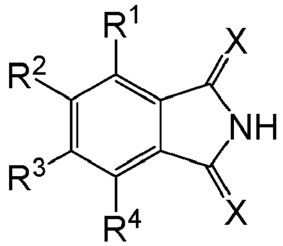

**IV**

The compounds of formula (**II**) are obtained from compounds of formula (**IV**).
Considering the extensive conjugated system present in these structures, these compounds have been shown to exhibit low energy band gaps between the HOMO (Highest-Occupied-Molecular-Orbitals) and LUMO (Lowest-Occupied-Molecular-Orbitals) which is responsible for their characteristic fluorescence properties (photoactivity) and electronic conduction (semi-conductor properties).

**Brief description of drawings**

For easier understanding of this application, figures are attached representing the preferred forms of implementation, which nevertheless are not intended to limit the technique disclosed herein.

Figure 1: Spectroeectrochemical experiments of (1) 1 mM in a solution of MeCN with 0.1 M TBAP, in a 3-electrode configuration spectroeletrochemical cell, WE platinum grid, CE platinum wire, reference electrode Ag/AgCl. Applied Potential -1.5 V and the spectrum measured during 15, 210, 600, 900, 1200, 1800, 2100, 2700, 3000 and 3600 s.

Figure 2: The reorganization energies were studied for the following compounds (**1**), (**4**), (**7**) - (**9**).

Figure 3: Reorganization Energies $\lambda^h$ (white bars) and $\lambda^e$ (black bars) calculated at the B3LYP/6-31+G(d,p) theory level and the relative mobilities $k^e/k^h$ calculated for T=300 K, neglecting differences in the donor/acceptor electronic coupling matrix elements.

**Detailed description**

The present application relates to compounds of formula (**I**) and (**II**), methods to produce the compounds and their use as components for optoelectronic devices.
The novel compounds of formula (**I**) are obtained from simple o-haloaryl amide acetals via a reaction sequence that involves treatment with iodine in acetone at reflux followed by further steps in the presence of a palladium catalyst, as seen in the example for the synthesis of compounds (**1**) - (**3**) given in Scheme 1.

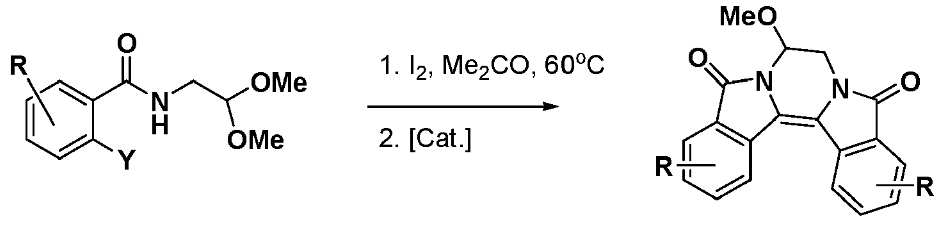


**(1)** Y = I, R = H (16% overall yield*)
**(1)** Y = Br, R = H (8% overall yield*)
**(2)** Y = Br, R = 4-Me (12% overall yield*)
**(3)** Y = Br, R = 5-Me (12% overall yield*)
* 2 steps

Scheme 1

The novel synthesis of (**I**) from (**III**) is achieved using an appropriate transition metal catalyst, preferable with Pd, however others like Rh, Ir or Cu could be used.

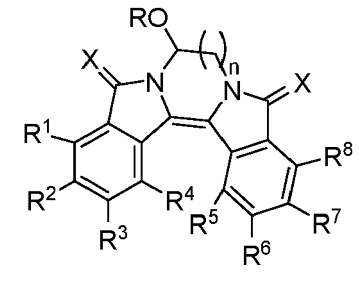

(I)

Compounds (**I**) wherein: X is O, S or Se;

n = 1-4;
R to $R^9$ represent: H, alkyl, aryl, vinyl, allyl, alkoxyl, OH, CN, CHO, $CO_2H$, $CO_2R$ where R represents any alkyl or aryl group, halogen: F, Br, Cl;
wherein in the case of $R^1$ to $R^8$ the adjacent pairs, including the pair R and $R^9$ can represent fused aromatic or cycloalkyl units.

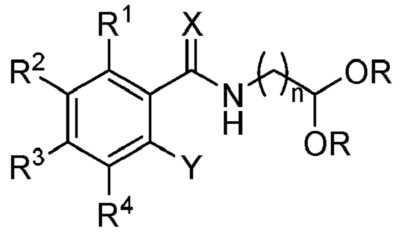

III

Compound (**III**) wherein Y is selected from a halogen, triflate or alkylsulfonates; $R^{1-4}$ are H, alkyl, aryl, vinyl, allyl, alkoxyl, OH, CN, CHO, $CO_2H$, $CO_2R$ where R represents any alkyl or aryl group, halogen: F, Br, Cl; and R is an alkyl or aryl group, and X is O, S or Se; and n = 1-3.

This two-step reaction normally is conducted first by the treatment of **(III)** with iodine and acetone in a flask fitted with a reflux condenser. After work-up, the crude reaction mixture is treated with the appropriate catalyst, in a flask under an inert gas atmosphere in the presence of a base and an appropriate solvent. In the case of the Pd catalyzed reactions, generally the following commercially available pre-catalysts are used: 1,1'-[bis(diphenylphosphino)ferrocene]dichloropalladium ($PdCl_2$ (dppf)), $PdCl_2$ $(CH_3CN)_2$, $Pd(CH_3CN)_4(BF_4)_2$, $PdCl_2$ $(PPh_3)_2$, palladium acetate ($(Pd(OAc)_2)$) and bis(dibenzylideneacetone)palladium ($(Pd(dba)_2)$).

The quantity of pre-catalyst varies between 5 and 30 mol%.

An appropriate ligand (preferably a phosphine ligand) is also added with the catalyst addition in order to form the active catalyst *in situ.* Iodine can be used from 1 to 30 mol% and acetone from 5 to 1000 ml.

Preferably, the *in situ* formed transition metal catalyst used is achiral.

In other embodiments, when appropriate chiral ligands are used, chiral catalysts can also be formed *in situ.*

Non chiral or racemic commercially available ligands like phosphane-derivatives can be included: triphenylphosphine ($PPh_3$), (±)2,2'-bis(diphenylphosphino)-1,1'-binaphthyl ((±)BINAP), tricyclohexylphosphine ($PCy_3$), tri(o-tolyl)phosphine (P(*o*-Tol)$_3$), tricyclohexylphosphine tetrafluoroborate ($Pcy_3.HBF_4$), tri-tert-butylphosphine (P(*t*-BU)$_3$), 4,5-bis(diphenylphosphino)-9,9-dimethylxanthene (Xantphos), (oxydi-2,1-phenylene)bis(diphenylphosphine) (DPEPhos), 1,1'-ferrocenediyl-bis(diphenylphosphine) (dppf), bis(diphenylphosphino)methane (dppm), 1,2-bis(dimethylphosphino)ethane (dmpe), 1,3-bis(diphenylphosphino)propane (dppp), 1,4-bis(diphenylphosphino)butane (dppb), 1,2-bis(diphenylphosphino)ethane (dppe), 1,3-bis(dicyclohexylphosphino)ethane (dcpe), Buchwald-type ligands like: 2-dicyclohexylphosphino-2'-(N,N-dimethylamino)biphenyl (DavePhos), 2-dicyclohexylphosphino-2',4',6'-triisopropylbiphenyl (Xphos), 2-dicyclohexylphosphino-2',6'-dimethoxybiphenyl (Sphos), 2-dicyclohexylphosphino-2',6'-diisopropoxybiphenyl (RuPhos), 2-(dicyclohexylphosphino)3,6-dimethoxy-2',4',6'-triisopropyl-1,1'-biphenyl (BrettPhos), etc. In the case of the chiral ligands; (+)-BINAP, (-)-BINAP, 2,2'-bis(di-p-tolylphosphino)-1,1'-binaphthyl (TolBINAP), 2,3-O-isopropylidene-2,3-dihydroxy-1,4-bis(diphenylphosphino)butane (DIOP), bis[(2-methoxyphenyl)phenylphosphino]ethane (DIPAMP), 5,6-bis((diphenylphosphanyl)methyl)-2,3-dimethoxy-2,3-dimethyl[1,4]dioxane (DioxPhos), *N*-benzyl-3,4-bis(diphenylphosphino)pyrrolidine (DeguPhos), 2,2',6,6'-tetramethoxy-4,4'-bis(di(3,5-xylyl)phosphino)-3,3'-bipyridine (Xylyl-P-Phos), (R)-(-)-4,12-bis(diphenylphosphino)-[2.2]-paracyclophane ((R)-Phanephos), (11a*R*)-(+)-10,11,12,13-tetrahydrodiindeno[7,1-de:1',7'-fg][1,3,2]dioxaphosphocin-5-phenoxy ((*R*)-ShiP), (11a*R*)-(+)-10,11,12,13-tetrahydrodiindeno[7,1-de:1',7'-fg][1,3,2]dioxaphosphocin-5-dimethylamine ((R)-SIPHOS), (2*R*,3*R*)-(+)-2,3-bis(diphenylphosphino)butane ((*R,R*)-Chiraphos), bis(diphenylphosphino)-7,8-dihydro-6H-dibenzo[f,h][1,5]dioxonin ($C_3$-TunePhos), (1,1',2,2')-1,1'-di-tert-butyl-(2,2')-diphospholane (TangPhos), (3,3',4,4',11b,11'b)-4,4'-di-t-butyl-4,4',5,5'-tetrahydro-3,3'-bi-3H-dinaphtho[2,1-c:1',2'-e]phosphepin (BINAPINE), 1,2-bis[4,5-dihydro-3H-binaphtho(1,2-c:2',1'-e)phosphepino]benzene (BINAPHANE), 4,12-bis(diphenylphosphino)-[2.2]-paracyclophane (PhanePhos), [(5,6),(5',6')-bis(ethylenedioxy)biphenyl-2,2'-diyl]bis(diphenylphosphine) (Synphos), (1,1',2,2')-2,2'-di-tert-butyl-2,3,2',3'-tetrahydro-1H,1'H(1,1')biisophosphindolyl (DuanPhos), 1-{(P)-2-[2-(diphenylphosphino)phenyl]ferrocenyl}ethyldicyclohexylphosp hine (Walphos) and 1-[(P)-2-(diphenylphosphino)ferrocenyl]ethyldicyclohexylphosphine (Josiphos) .

The quantity of ligand varies between 5 and 30 mol%.

In the case of the base the following are generally used: triethylamine, $K_2CO_3$, $CaCO_3$, $Ba(OH)_2$, N,N-diisopropylethylamine (DIPEA), $K_3PO_4$, N-methylmorpholine (NMM), 1,8-Diazabicyclo[5.4.0]undec-7-ene(DBU), KOH, KF and $Cs_2CO_3$. The quantity of base used ranges from 0.5 to 3 equivalents.

The following reaction solvents can be used: toluene, dimethyl ether, diethyl ether, dichloroethane (DCE), tetrahydrofuran (THF), 1,4-dioxane, acetone, acetonitrile, MeTHF, 1,2-dichloroethane, dimethylformamide (DMF), dimethoxyethane (DME), dimethylacetamide (DMA) and N-methylpiperidine (NMP), used in quantities that vary from 1 to 1000 mL.

The reactions are generally run at temperatures of between 60°C and 120°C. The reaction times varies between 10 and 24 hours.

Some examples are shown in Table 1.

**Table 1.** Synthesis of a compound of Formula (**I**) (compound (**1**)): reaction screening.

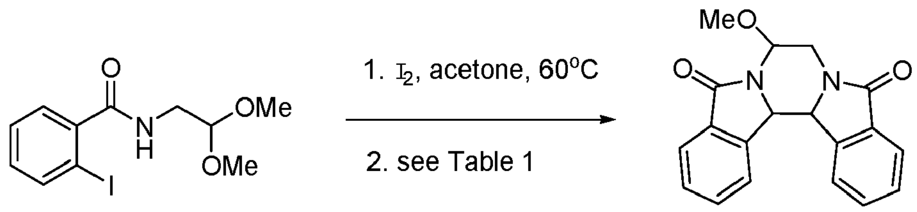


(III) (I)

| Entry[a] | [Cat] | Base | Solvent | Temp. /°C | two-step yield/% |
|---|---|---|---|---|---|
| **1** | $PdCl_2$ (dppf) | $Cs_2CO_3$ | THF | 100 | 11 |
| **2** | $PdCl_2$ $(PPh_3)_2$ | | | | 15 |
| **3[b]** | $Pd(OAc)_2/PPh_3$ | | | | 12 |
| **4[b]** | $Pd(dba)_2/PPh_3$ | | | | 13 |
| **5** | $PdCl_2$ $(PPh_3)_2$ | $K_3PO_4$ | | | 16 |
| **6[b]** | $Pd(OAc)_2/PPh_3$ | $K_2CO_3$ | | | 8 |
| **7[b]** | | $NEt_3$ | | | 16 |
| **8[b]** | | $K_3PO_4$ | | | 17 |
| **9[b]** | | DIPEA | | | 12 |
| **10[b]** | | $K_3PO_4$ | $CH_3CN$ | | 7 |
| **11[b]** | | | MeTHF | | 9 |
| **12[b]** | | | DME | | 10 |
| **13[b]** | | | DCE | | 3 |
| **14[b]** | | | 1,4-Dioxane | 120 | 13 |
| **15[b]** | | | Toluene | 120 | 7 |

[a]Reaction conditions: 20 mol% [Pd], 3 equivalents base, 24h. [b]30 mol% of $PPh_3$ was used.

The compounds of formula (**II**) are obtained from appropriate phthalimide precursor (**IV**) in quantities that range from 0.5 to 250 moles using a sequence that first involves coupling with an appropriate linker in quantities that range from 1 to 3 equivalents to form a di-phthalimide intermediate (Krchová et al. 2013) - followed by a McMurry coupling (Hassner and Stumer, 1994) to form the final compound. An example is the synthesis of compound (**4**) given in Scheme 2.

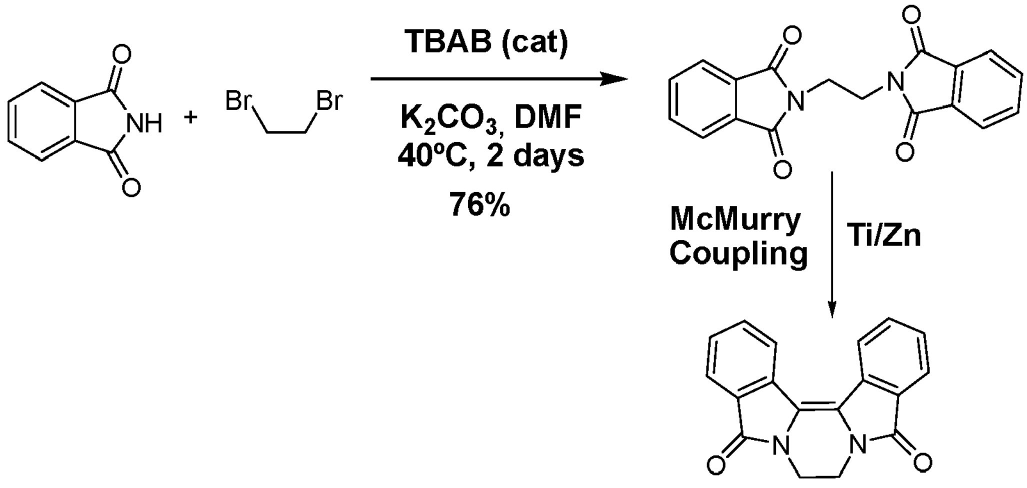


**4**

Scheme 2

In fact, the pentacyclic compound described in Scheme 2, which is highly fluorescent, is a known fluorescence standard that was used for the development of an analytical protocol for detecting the presence of 7,8-diaminopelargonic acid aminotransferase (Mann *et al.* 2013).

The novel synthesis of **(II)** from **(IV)** is achieved in a two-step reaction sequence. The first reaction, to synthesize the di-phthalimide intermediate involves the use of a literature procedure with a base in quantities that range from 1 to 4 molecular equivalents and solvent in quantities that vary from 1 to 1000 mL, however other bases like NaH could be used. Other polar solvents also could be used, like toluene, DMA, DMSO, etc. The second step involves the well-known McMurry coupling, where titanium ($TiCl_4$) and zinc (Zn) were used as reagents to achieve compounds of formula **(II).** Titanium ($TiCl_4$) can be used from 1 to 10 molecular equivalents and zinc (Zn) can be used from 2 to 20 equivalents.

The linker in compounds of formula **(II)** is obtained from: 1,2-dibromoethane, 1,2-dichloroethane, 1,2-diiodoethane, 1,3-dibromopropane, 1,3-dichloropropane, 1,3-diiodoopropane, 1,4-dibromobutane, 1,4-dichlorobutane and 1,4-diiodobutane.

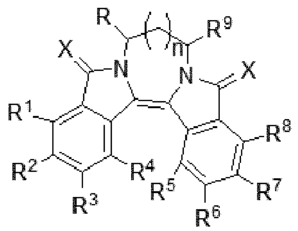

II

Compounds **(II)** wherein:

X is O, S or Se,
n = 0-3,
R to $R^9$ represent: H, alkyl, aryl, vinyl, allyl, alkoxyl, OH, CN, CHO, $CO_2H$, $CO_2R$ where R represents any alkyl or aryl group, halogen: F, Br, Cl;
wherein in the case of $R^1$ to $R^8$ the adjacent pairs, including the pair R and $R^9$ optionally represent fused aromatic or cycloalkyl units.

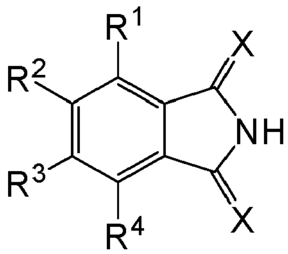

**IV**

Compound **(IV)** wherein:

$R^{1-4}$ represent H, alkyl, aryl, vinyl, allyl or alkoxyl, OH, CN, CHO, $CO_2H$ and $CO_2R$ where R represents any alkyl or aryl group, halogen: F, Br, Cl; and X is O, S or Se.

Literature procedures were used, but solvents like MeTHF, diethyl ether, $CH_3CN$ could also be used. Addition of bases, like pyridine, could also be an improvement of the method. Two other derivatives of **(1)** were synthesized, namely the mono-seleniated compound **(5)** and the di-sulphur compound **(6)** (Scheme 3).

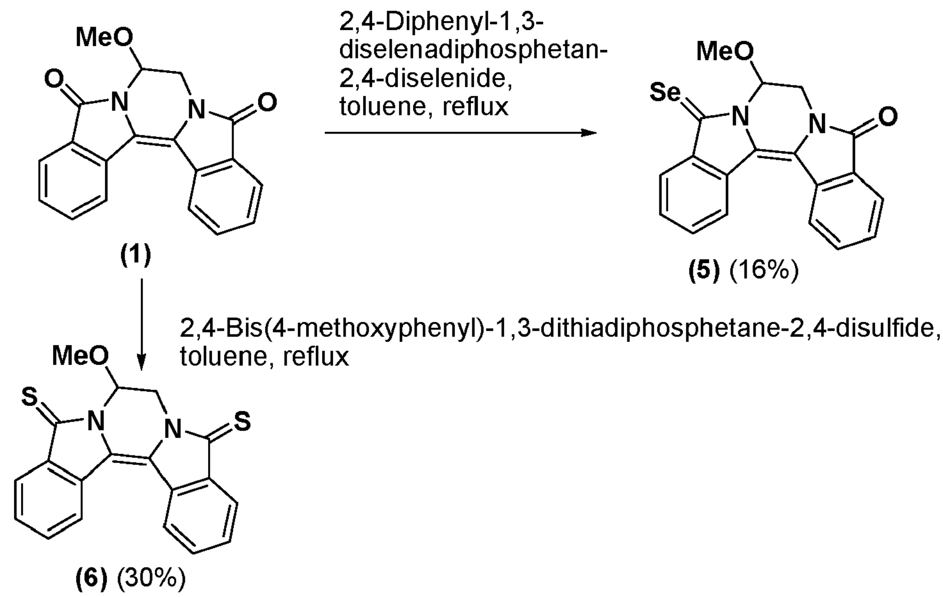


Scheme 3

The photoelectrochemical properties of the compounds were explored to determine their appropriateness for their application as optoelectronic devices.

All electrochemical properties were studied by cyclic voltammetry (CV) measurements.

Spectroelectrochemistry and UV-vis- spectra of (**1**) were studied and recorded in Acetonitrile (MeCN) plus 0.1 M tetrabutylammonium perchlorate (TBAP) under nitrogen flow, and the results are shown in Figure 1. Compound (**1**) and the unsubstituted one (**4**), both presented one chemically irreversible bielectronic oxidation wave with anodic peak potential $E_{pa}$ at 1.30 V and 1.27V, and both also presented two reversible/quasi reversible one-electron transfer reduction waves $E_{pc1}$ at -1.45 and -1.52 V and $E_{pc2}$ at -1.73 and -1.83 V (vs SCE), respectively. In the cathodic direct scan, at different scan rates, two successive reversible/quasi-reversible one electron transfer process, with a standard potential of -1.40 V and -1.44 V and -1.69 V and -1.77 V (vs. SCE) were observed, respectively.

In a conductor or semiconductor, it is usually the valence band (the highest occupied energy band) that is partially filled. That is, there is an overlap between the valence band and the conduction band (the lowest unoccupied energy band). Semiconductors usually have low energy gaps below 4.0 eV. In the present case the LUMO energies of (**1**) and the unsubstituted example (**2**) were estimated to be around -3.29 eV and -3.28 eV and HOMO presented a value

of -5.89 eV and -5.82 eV by cyclic voltammetry, (using the ferrocene as reference to calculate the Homo ($E_{Homo}$= -e [$E_{oxoffset}$+4.4]) and Lumo ($E_{Lumo}$= -e[$E_{redoffset}$+4.4]) (Gawrys, et al. 2010).

Therefore, their corresponding electrochemical energy gaps (EgEC) were estimated to be 2.6 and 2.54 eV respectively, as described above, that could indicate that (**1**) and the unsubstituted compound (**4**) are semiconductor. The charged species play a critical role on the charge carriers of semiconducting heteroatom-doped (**1**) associated with the doped state. In the direct anodic scan, a chemical irreversible two-electron oxidation wave at 1.30 V and 1.27 V suggests that the charged species (radical cation and dication) were not stable at the measurement conditions.

**Table 2.** Electrochemical properties of compounds (**1**) and (**4**)

| Compounds | $E_{pc1}$ (V vs SCE) | $E^0_1$ (V vs SCE) | $E_{pc2}$ (V vs SCE) | $E^0_2$ (V vs SCE) | $E_{pa3}$ (V vs SCE) | HOMO (eV) | LUMO (eV) | EgEC (eV) |
|---|---|---|---|---|---|---|---|---|
| 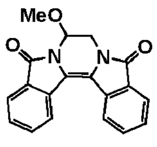 **1** | -1.45 | -1.40 | -1.73 | -1.69 | 1.30 | -5.89 | -3.29 | 2.60 |
| 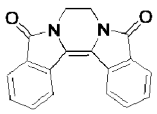 **4** | -1.52 | -1.44 | -1.83 | -1.77 | 1.27 | -5.82 | -3.28 | 2.54 |

All spectroelectrochemistry UV-vis-NIR spectra of (**1**) and (**4**) were studied and carried out in MeCN + 0.1 M TBAP under nitrogen flow. As shown in Figure 1, the initial spectrum of (**1**) and (**4**) showed two major absorption bands: a narrow band with a maximum at 280 nm and a broader band between 325-450 nm, respectively. Following the results of the cyclic voltammetry study just described, it was realized that there is the potential for the generation of the fluorescent and electrochromic radical anion and, therefore, a charge of - 1.5 V (vs. SCE) was applied for 3600 s. After just 15 s of this experiment the spectra changed, with the broad band (between 325-450 nm) starting to decrease and two new absorption bands appearing at 480 and 620 nm, respectively. The band at 250 nm shifts to 280 nm. These absorption bands increase over a 3000 s period. After 3600 s (in the case of (**1**)) and 360s (in the case of (**4**)) the bands decreased, probably due to the adsorption and precipitation of the material in the cell.

The absorption spectra for compounds (**1**) and (**4**) in chloroform, acetonitrile and ethanol are quite similar and contain three main absorption bands: around 280, 390 and 410 nm. A small shift to shorter wavelengths was observed as the polarity of the solvent increased, that is consistent with $n \rightarrow \pi^*$ electronic transitions (Rohatgi-Mukherjee, 1978). The emission spectra of both compounds are also very similar with a maximum around 465 nm. Compound (**1**) and (**4**) exhibit a bright blue luminescence with a high quantum yield of 0.56 and 0.60, respectively, determined by the comparative method using 7-(diethylamino)-4-methylcoumarin ($\phi$=**0.5)** **(**Taniguchi and Lindsey 2018).

Quantum chemical calculations were conducted for compounds (**1**), (**4**) and (**7**)-(**9**) and the structures are shown in Figure 2. Geometry optimization, accompanied by frequency analysis, were performed in neutral, cationic, dicationic, anionic and dianionic forms of the compounds at the B3LYP/6-31+G(d,p) theory level. The frequency analysis verified the optimized structures as energy minima.

The electron and hole transport in an organic solid can be considered as an electron/hole hopping process. A crucial factor to characterize the process is the reorganization energy for hole ($\lambda^h$) and electron ($\lambda^e$) transport, which measure the energy cost for carrier exchange. In the case of an n-type material, as the present compounds, a smaller $\lambda^e$ value reflects smaller energy cost in the hopping process and enhanced transport properties (Lin et al. 2003) . In order to compare the electron and hole mobilities, the relative hopping rates can be calculated by $\frac{k^e}{k^h} = (\lambda_e/\lambda_h)^{1/2} exp(-(\lambda_e-\lambda_h)/4k_BT)$, admitting that the differences in the donor/acceptor electronic coupling matrix elements can be neglected. The calculated values of the reorganization energies and the relative electron/hole mobilities are depicted in Figure 3.

While compound (**9**) presented the lowest reorganization energies, it was compound (**7**) that showed the most favorable electron/hole relative mobilities. It is to be expected that other related compounds of formula (**I**) and (**II**) should show even better electronic properties.

**Table 3.** Maximum absorption and emission of compound (**1**) ($\lambda_{exc}$=390 nm) and (**4**) ($\lambda_{axc}$=395 nm) in different solvents.

| | | Compound (1) | | | Compound (4) | | |
|---|---|---|---|---|---|---|---|
| Solvent | $E_T^N$ a) | $\lambda_{abs}$ (nm) | $\lambda_{em}$ (nm) | | $\lambda_{abs}$ (nm) | $\lambda_{em}$ (nm) | $\phi_F$ |
| chloroform | 0.259 | 396 | 467 | | 398 | 467 | |
| acetonitrile | 0.460 | 392 | 462 | | 396 | 468 | |
| ethanol | 0.654 | 392 | 461 | 0.56[b] | 395 | 464 | 0.60[b] |

a) normalized empirical parameters of solvent polarity

b) determined by the comparative method using 7-(diethylamino)-4-methylcoumarin (Coumarin 1) ($\phi$= 0.5) in ethanol as reference (Taniguchi and Lindsey, 2018).

**Examples**

**Synthetic Protocols**

**Synthesis of the compound (1)**

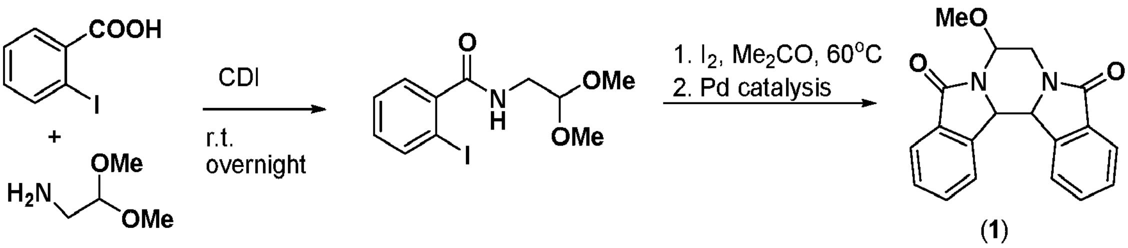


(**1**)

*Synthesis of N-(2,2-dimethoxyethyl)-2-iodobenzamide*

2-Iodobenzoic acid (5.0 g, 0.02 mol), THF (50 mL) and 1,1'-carbonyldiimidazole (CDI) (3.24 g, 0.02 mol) were added to a round-bottom flask. The mixture was left stirring at a temperature between 20 and 30°C for 30 min. After that, 2,2-dimetoxyethanamine (2.18 mL, 0.02 mol) was slowly added to the reaction flask. The reaction was left stirring between 20 and 30°C overnight. The solvent was evaporated under reduced pressure and after purification by column chromatography with silica gel, using Hexane/ EtOAc (1/1), the title compound was obtained as a white oily solid (6.50 g, 98% yield).

$^1H$ NMR (400 MHz, $CDCl_3$) $\delta$: 3.41 (s, 6H, 2×Ome), 3.58 (d, 2H, $CH_2$, J= 5.2 Hz), 4.53 (t, 1H, CH, J= 5.2 Hz), 6.02 (s br, 1H, NH), 7.06-7.10 (m, 1H, Ar), 7.34-7.36 (m, 2H, Ar), 7.83-7.86 (m, 1H, Ar). $^{13}C$ NMR (100 MHz, $CDCl_3$) $\delta$: 41.3, 53.3, 92.2, 102.0, 128.6, 131.0, 139.1, 143.2, 169.6.

*Synthesis of 4'-hydroxy-4-methoxy-3,4-dihydro-1H-2,3'-biisoquinoline-1,1' (2'H) -dione* **(1)**:

A mixture of N-(2,2-dimethoxyethyl)-2-iodobenzamide (1 g, 3 mmol), and iodine (76 mg, 0.3 mmol) in acetone (15 ml) was stirred under reflux overnight. Most of the acetone was then removed by evaporation under reduced pressure and the residue was diluted with $CH_2Cl_2$ (20 ml). The mixture was washed successively with 5% aq. $Na_2S_2O_3$ (10 ml), $H_2O$ (20 ml) and brine (20 ml). The organic layer was separated, dried over $MgSO_4$ and filtered. The solvent was removed under reduced pressure. The crude product was passed through a glass column filled with silica gel and eluted with $Et_2O$. The major fraction was recovered and evaporated under reduced pressure to afford the "pre-purified" crude product that was dissolved in dry THF (20 ml). $PdCl_2(PPh_3)_2$ (0.18 g, 20 mol%) and $Cs_2CO_3$ (1.3 g, 3 equiv.) were then added to the reaction flask, under an inert atmosphere. The reaction was stirred at 100°C for 18 hours. The solvent was evaporated under reduced pressure and the crude product dissolved in $CH_2Cl_2$ (20 ml) and washed with aqueous saturated $NaHCO_3$ solution (20 ml), $H_2O$ (20 ml) and brine (20 ml). The organic layers were dried with $MgSO_4$, filtered

and the solvent evaporated under reduced pressure. The crude product was purified by silica gel column chromatography using hexane/$Et_2O$ (1:1) as eluent to afford the title compound (**1**) as a yellow solid (0.078 g, 16% overall two-steps yield).

$^1H$ NMR (400 MHz, $CDCl_3$) δ: 3.42 (s, 3H, $OCH_3$), 3.53 (dd, J= 16 Hz, 1H, $CH_2$), 4.74 (dd, J= 16 Hz, 1H, $CH_2$), 5.93-5.94 (m, 1H, CH), 7.52-7.59 (m, 2H, Ar), 7.68-7.76 (m, 2H, Ar), 7.95 (d, J= 8 Hz, 1H, Ar), 8.01 (d, J= 8 Hz, 1H, Ar), 8.20-8.26 (m, 2H, Ar). $^{13}C$ NMR (100 MHz, $CDCl_3$) δ: 42.7, 56.9, 75.6, 117.8, 120.9, 122.7, 124.6, 125.1, 129.1, 129.4, 130.2, 132.6, 132.9, 133.5, 165.2, 165.4. HR-MS (ESI) m/z Calcd for $C_{19}H_{15}N_2O_3$ ($M^+$-$H_2O$) 319.10827, Found 319.10772 ($M^+$-$H_2O$) for $C_{19}H_{15}N_2O_3$.

**Synthesis of the compound (2)**

Synthesis of *2-bromo-N-(2,2-dimethoxyethyl)-4-methylbenzamide*

2-Bromo-4-methylbenzoic acid (1.0 g, 4.7 mmol), THF (30 mL) and CDI (0.75 g, 4.7 mmol) were added to a round-bottom flask. The mixture was left stirring between 20 and 30°C for 30 min. After that, 2,2-dimetoxyethanamine (0.51 mL, 4.7 mmol) was slowly added to the reaction flask. The reaction was left stirring between 20 and 30°C overnight. The solvent was evaporated under reduced pressure and after purification by column chromatography with silica gel, using Hexane/EtOAc (1/1), the title compound was obtained as a white oil/solid (1.134 g, 80% yield).

$^1H$ NMR (400 MHz, DMSO-d6) δ: 2.31 (s, 3H, $CH_3$), 3.30-3.35 (m,8H, 2xOMe and $CH_2$), 4.49 (t, J=5.2Hz, 1H, CH), 7.21-7.25 (m, 2H, ArH), 7.48 (s br, 1H, ArH), 8.44 (s br, 1H, NH). $^{13}C$ NMR (100 MHz, DMSO-*d*6) δ: δ 20.8, 41.3, 53.6, 102.2, 119.2, 128.5, 129.1, 133.4, 136.5, 141.4, 167.9.

Synthesis of 7-methoxy-2,13-dimethyl-7,8-dihydropyrazino[2,1-a:3,4-a']diisoindole-5,10-dione **(2):**

A mixture of 2-bromo-N-(2,2-dimethoxyethyl)-4-methylbenzamide (1.134 g, 3.75 mmol), and iodine (105 mg, 0.375 mmol) in acetone (15 ml) was stirred at reflux temperature, 60°C, overnight. Most of the acetone was then removed by evaporation under reduced pressure and the residue was diluted with $CH_2Cl_2$ (20 ml). The mixture was washed successively with 5% aq. $Na_2S_2O_3$ (10 ml), $H_2O$ (20 ml) and brine (20 ml). The organic layer was separated, dried over $MgSO_4$ and filtered. The solvent was removed under reduced pressure. The crude product was passed through a glass column filled with silica gel and eluted with $Et_2O$. The major fraction was recovered and evaporated under reduced pressure to afford the "pre-purified" crude product that was dissolved in dry THF (20 ml). $Pd(OAc)_2$ (39 mg, 20 mol%), $PPh_3$ (68 mg, 30 mol%) and $K_3PO_4$ (549 mg, 3 equiv.) were then added to the reaction flask, under an inert atmosphere. The reaction was stirred at 100°C for 18 hours. The solvent was evaporated under reduced pressure and the crude product dissolved in $CH_2Cl_2$ (20 ml) and washed with aq. sat. $NaHCO_3$ (20 ml), $H_2O$ (20 ml) and brine (20 ml). The organic layers were dried with $MgSO_4$, filtered and the solvent evaporated under reduced pressure. The crude product was purified by silica gel column chromatography using hexane/$Et_2O$ (1:1) as eluent to afford the title compound **(2)** as a yellow solid (150.6 mg, 12% overall two-steps yield) . $^1H$ NMR (400 MHz, $CDCl_3$) δ: 2.58 (s, 3H, CH3), 2.60 (s, 3H, CH3), 3.40 (s, 3H, $OCH_3$), 3.48-3.52 (dd, 1H, $CH_2$), 4.69-4.72 (d, 1H, $CH_2$), 5.90 (s, 1H, CH), 7.35-7.40 (m, 2H, Ar), 7.83-7.90 (m, 2H, Ar), 8.01-8.05 (m, 2H, Ar). $^{13}C$ NMR (100 MHz, $CDCl_3$) δ: 22.55, 22.63, 42.86, 56.79, 75.59, 117.88, 121.01, 123.14, 123.29, 124.28, 124.84, 126.79, 127.88, 130.47, 130.49, 133.73, 134.02, 142.98, 143.74, 165.34, 165.57. MS (ESI-TOF) m/z: 347.21 ($M^+$).

**Synthesis of the compound (3)**

Synthesis of *2-bromo-N-(2,2-dimethoxyethyl)-5-methylbenzamide*

2-Bromo-5-methylbenzoic acid (1.0 g, 4.7 mmol), THF (30 mL) and 1,1'-carbonyldiimidazole (0.75 g, 4.7 mmol) were added to a round-bottom flask. The mixture was left stirring between 20 and 30°C for 30 min. After that, 2,2-dimetoxyethanamine (0.51 mL, 4.7 mmol) was slowly added to the reaction flask. The reaction was left stirring between 20 and 30°C overnight. The solvent was evaporated under reduced pressure and after purification by column chromatography with silica gel, using Hexane/EtOAc (1/1), the title compound was obtained as a white oil/solid (1.25 g, 88% yield). $^1H$ NMR (400 MHz, DMSO-d6) δ: 2.28 (s, 3H, $CH_3$), 3.30 (s br, 2H, $CH_2$), 3.35 (s, 6H, 2xOMe), 4.48-4.51 (m, 1H, CH), 7.17-7.21 (m, 2H, Ar), 7.50 (d, J = 8.0 Hz, 1H, Ar), 8.48 (s br, 1H, NH). $^{13}C$ NMR (100 MHz, DMSO-d6) δ: 20.7, 41.3, 53.6, 102.1, 116.0, 129.7, 131.9, 132.9, 137.6, 139.2, 167.9.

Synthesis of 7-methoxy-3,12-dimethyl-7,8-dihydropyrazino[2,1-a:3,4-a']diisoindole-5,10-dione **(3):**

A mixture of *2-bromo-N-(2,2-dimethoxyethyl)-5-methylbenzamide* (1.25 g, 4.15 mmol), and iodine (105 mg, 0.415 mmol) in acetone (15 ml) was stirred at reflux temperature, 60°C, overnight. Most of the acetone was then removed

by evaporation under reduced pressure and the residue was diluted with $CH_2Cl_2$ (20 ml). The mixture was washed successively with 5% aq. $Na_2S_2O_3$ (10 ml), $H_2O$ (20 ml) and brine (20 ml). The organic layer was separated, dried over $MgSO_4$ and filtered. The solvent was removed under reduced pressure. The crude product was passed through a glass column filled with silica gel and eluted with $Et_2O$. The major fraction was recovered and evaporated under reduced pressure to afford the "pre-purified" crude product that was dissolved in dry THF (20 ml). $Pd(OAc)_2$ (42 mg, 20 mol%), $PPh_3$ (72 mg, 30 mol%) and $K_3PO_4$ (584 mg, 3 equiv.) were then added to the reaction flask, under an inert atmosphere. The reaction was stirred at 100°C for 18 hours. The solvent was evaporated under reduced pressure and the crude product dissolved in $CH_2Cl_2$ (20 ml) and washed with aq. sat. $NaHCO_3$ (20 ml), $H_2O$ (20 ml) and brine (20 ml). The organic layers were dried with $MgSO_4$, filtered and the solvent evaporated under reduced pressure. The crude product was purified by silica gel column chromatography using hexane/$Et_2O$ (1:1) as eluent to afford the title compound **(3)** as a yellow solid (170.2 mg, 12% overall two-step yield).

$^1H$ NMR (400 MHz, $CDCl_3$) δ: 2.49 (s, 3H, CH3), 2.51 (s, 3H, $CH_3$), 3.41 (s, 3H, $OCH_3$), 3.49-3.53 (dd, 1H, $CH_2$), 4.70-4.73 (d, 1H, $CH_2$), 5.91 (s, 1H, CH), 7.47-7.54 (m, 2H, Ar), 7.74-7.79 (m, 2H, Ar), 8.03-8.09 (m, 2H, Ar). $^{13}C$ NMR (100 MHz, $CDCl_3$) δ: 21.50, 21.67, 42.85, 56.86, 75.64, 117.41, 120.46, 122.51, 122.55, 124.73, 125.20, 129.37, 130.46, 130.73, 131.07, 133.23, 133.98, 139.89, 140.00, 165.43, 165.63. MS (ESI-TOF) m/z: 347.18 ($M^+$).

*Synthesis of 8-methoxy-10-selenoxo-7,8-dihydropyrazino[2,1-a:3,4-a']diisoindol-5(10H)-one* **(5)**

A mixture of 4'-hydroxy-4-methoxy-3,4-dihydro-1*H*-2,3'-biisoquinoline-1,1'(2'H)-dione **(1)** (100 mg, 0.31 mmol), and 2,4-diphenyl-1,3-diselenadiphosphetane 2,4-diselenide (Woollin's Reagent) (167 mg, 0.31 mmol) in toluene (5 ml) was stirred at reflux temperature, 112°C, overnight. Most of the toluene was then removed by evaporation under reduced pressure and the crude mixture was purified by silica gel column chromatography using hexane, hexane/AcOEt (9/1), (5/1), (1/1) and AcOEt as eluents to afford the title compound **(5)** as a dark red solid (18 mg, 16% yield).

$^1H$ NMR (400 MHz, $CDCl_3$) δ: 3.44 (s, 3H, OMe), 3.70-3.74 (d, J= 16 Hz, 1H, $CH_2$), 5.50-5.54 (d, J= 16 Hz, 1H, $CH_2$), 6.00-6.01 (m, 1H, CH), 7.52-7.56 (t, J= 8 Hz, 1H, Ar), 7.67-7.80 (t, J= 8 Hz, 2H, Ar), 8.03-8.05 (d, J= 8 Hz, 1H, Ar), 8.22-8.24 (d, J= 8 Hz, 2H, Ar), 8.40-8.42 (d, J= 8 Hz, 1H, Ar). $^{13}C$ NMR (100 MHz, $CDCl_3$) δ: 49.48, 57.11, 75.89, 119.34, 121.74, 123.49, 125.76, 126.11, 127.25, 129.33, 129.63, 130.41, 130.82, 131.77, 133.57, 133.91, 140.70, 165.31, 188.43. MS (ESI-TOF) m/z: 383.03 ($M^+$).

*Synthesis of 7-methoxy-7,8-dihydropyrazino[2,1-a:3,4-a']diisoindole-5,10-dithione* **(6)**

A mixture of 4'-hydroxy-4-methoxy-3,4-dihydro-1*H*-2,3'-biisoquinoline-1,1'(2'*H*)-dione **(1)** (104 mg, 0.33 mmol), and 2,4-bis-(4-methoxyphenyl)-1,3-dithia-2,4-diphosphetane 2,4-disulfide (Lawesson's Reagent) (264 mg, 0.65 mmol) in toluene (20 ml) was stirred at reflux temperature, 112°C, overnight. Most of the toluene was then removed by evaporation under reduced pressure and the residue was diluted with AcOEt (20 ml). The mixture was washed successively with $H_2O$ (20 ml) and brine (20 ml). The organic layer was separated, dried over $MgSO_4$ and filtered. The solvent was removed under reduced pressure. The crude product was purified by silica gel column chromatography using hexane/$Et_2O$ (5/1) and $Et_2O$ as eluents to afford the title compound **(6)** as a dark orange solid (36 mg, 30% yield).

$^1H$ NMR (400 MHz, $CDCl_3$) δ: 3.51 (s, 3H, OMe), 3.77-3.81 (d, 1H, $CH_2$), 5.45-5.54 (d, 1H, $CH_2$), 6.68 (s, 1H, CH), 7.60-7.66 (m, 2H, Ar), 7.73-7.80 (m, 2H, Ar), 8.17-8.24 (m, 2H, Ar), 8.30-8.36 (m, 2H, Ar). $^{13}C$ NMR (100 MHz, $CDCl_3$) δ: 46.52, 57.93, 77.77, 122.22, 122.31, 124.90, 125.98, 126.65, 128.71, 130.12, 130.16, 131.69, 131.97, 132.03, 132.67, 137.11, 137.60, 187.06, 187.72. MS (ESI-TOF) m/z: 351.14 ($M^+$).

**Synthesis of the compounds of formula (II)**

**Synthesis of 7,8-dihydropyrazino[2,1-a:3,4-a']diisoindole-5,10-dione (4)**

*Synthesis of 2,2'-(ethane-1,2-diyl)bis(isoindoline-1,3-dione) :*

Phthalimide (1.0 g, 6.8 mmol), TBAB (0.219 g, 0.68 mmol), $K_2CO_3$ (1.9 g, 13.6 mmol), 1,2-dibromoethane (1.0 mL, 12 mmol) and DMF (15 mL) were added to a round-bottom flask. The mixture was left stirring at 40°C for 2 days. After that, the solvent was evaporated under reduced pressure and the crude mixture diluted with AcOEt and water. The aqueous layer was washed with AcOEt (3 × 30 mL). The combined organic layers were dried with $MgSO_4$, filtered and the solvent evaporated under reduced pressure. The title compound was obtained as a white solid (1.28 g, 27% yield). $^1H$ NMR (400 MHz, $CDCl_3$) δ: 4.00 (s, 4H, $CH_2$), 7.67-7.70 (m, 4H, Ar), 7.74-7.79 (m, 4H, Ar). $^{13}C$ NMR (100 MHz, $CDCl_3$) δ: 36.9, 123.5, 132.0, 134.1, 168.3.

*Synthesis of 7,8-dihydropyrazino[2,1-a:3,4-a']diisoindole-5,10-dione* **(4)**

Zn powder (1.6 g, 24 mmol) was stirred at 0°C in THF (40 mL). $TiCl_4$ (1.3 mL, 12 mmol) was slowly added to the reaction flash and the mixture stirred between 20 and 30°C for 1 hour and at reflux 3 hours. After that the reaction was cooled to 0°C and pyridine (0.5 mL, 6 mmol) was added to the reaction flask. The reaction was stirred 10 minutes at 0°C. After that 2,2'-(ethane-1,2-diyl)bis(isoindoline-1,3-dione) (0.8 g, 2.4 mmol) in THF (15 mL) was added slowly to the reaction flask. The reaction mixture was refluxed overnight and monitored by TLC. The reaction was quenched with $K_2CO_3$ (10% aq. Solution) and $CH_2Cl_2$ was added to the reaction flask. The mixture was extracted three times with $CH_2Cl_2$. The combined organic layers were dried with $MgSO_4$, filtered and the solvent evaporated under reduced pressure. The crude product was purified by silica gel column chromatography using $CHCl_3$/AcOEt (5:1) and (1:1) as eluents to afford the title compound **(4)** as a yellow solid (0.1 g, 16% yield) . $^1$H NMR (DMSO, 400 MHz) δ: 4.02 (s, 4H, $CH_2$), 7.61-7.65 (t, J= 8 Hz, 2H, Ar), 7.78-7.82 (t, *J*=8 Hz, 2H, Ar), 7.88-7.90 (d, J= 8 Hz, 2H, Ar), 8.35-8.37 (d, J= 8 Hz, 2H, Ar). $^{13}$C NMR (DMSO, 100 MHz) δ: 37.1, 119.9, 123.0, 123.7, 129.3, 129.4, 132.7, 132.8, 163.8. HR-MS (ESI) m/z Calcd for $C_{18}H_{12}N_2O_2$ 288.0899 ($M^+$), Found 289.0973 ($M^+$)for $C_{18}H_{13}N_2O_2$.

***Electrochemical measurements:***

**3-electrode configuration Cyclic Voltammetry:**

Cyclic voltammetry (CV), measurements were performed on an Autolab PGSTAT 12 potentiostat/galvanostat, controlled with GPES software version 4.9 (Eco-Chemie), using a cylindrical 3-electrode cell of 5mL. A glassy carbon electrode or platinum (EDAQ) was used as the working electrode and a Pt wire was used as an auxiliary electrode. All potentials refer to an Ag/AgCl leakless (3M KCl) reference electrode (EDAQ). Prior to use, the working electrode was polished in aqueous suspensions of 1.0 and 0.3 mm alumina (Beuhler) over 2-7/8" micro-cloth (Beuhler) polishing pads, then rinsed with distilled water and methanol. This cleaning procedure was always applied before any electrochemical measurements. Electrochemical cleaning was also employed, submitting the electrode to -1/+ 1 V currents for 60 s. Cyclic voltammetry (CV) at a scan rate of 20, 50 and 100 mV/s was used to characterize the electrochemical responses in the desired electrochemical window.

Spectroelectrochemical experiments were performed in a thin layered quartz glass cell using platinum gauze and platinum wire as working and counter electrodes respectively, whereas an Ag/AgCl was used as a reference electrode. Autolab PGSTAT 12 potentiostat/galvanostat, controlled with GPES software version 4.9 (Eco-Chemie) and UV-Vis spectra were acquired with a Shimadzu UV 2501 PC or with a Varian Cary 100 Bio spectrophotometers.

Fluorescence spectra were acquired on a SPEX Fluorolog-3 Model FL3-22 spectrofluorimeter.

**Abbreviations**

| | |
|---|---|
| BINAP | (1,1'-Binaphthalene-2,2'-diyl)bis(diphenylphosphine) |
| BrettPhos | 2-(Dicyclohexylphosphino)3,6-dimethoxy-2',4',6'-triisopropyl-1,1'-biphenyl |
| Bu | butyl |
| CDI | Carbonyl diimidazole |
| Cy | cyclohexyl |
| DavePhos | 2-Dicyclohexylphosphino-2'-(N,N-dimethylamino)biphenyl |
| dba | dibenzylideneacetone |
| DBU | 1,8-Diazabicyclo[5.4.0]undec-7-ene |
| dcpe | 1,2-Bis(dicyclohexylphosphino)ethane |
| DIPEA | N,N-Diisopropylethylamine |
| DMA | Dimethylacetamide |
| DME | Dimethoxyethane |
| DMF | Dimethylformamide |
| dmpe | 1,2-Bis(dimethylphosphino)ethane |
| DPEPhos | (Oxydi-2,1-phenylene)bis(diphenylphosphine) |
| dppb | 1,4-Bis(diphenylphosphino)butane |
| dppe | Ethylenebis(diphenylphosphine) |
| dppf | 1,1'-Bis(diphenylphosphino)ferrocene |
| dppm | Bis(diphenylphosphino)methane |
| dppp | 1,3-Bis(diphenylphosphino)propane |

| | |
|---|---|
| DMSO | Dimethylsulfoxide |
| NMM | 4-Methylmorpholine |
| NMP | Methylpyrrolidone |
| Py | pyridine |
| RuPhos | 2-Dicyclohexylphosphino-2',6'-diisopropoxybiphenyl |
| Sphos | 2-Dicyclohexylphosphino-2',6'-dimethoxybiphenyl |
| TBAB | Tetrabutylammonium bromide |
| THF | Tetrahydofuran |
| Tol | tolyl |
| XantPhos | 4,5-Bis(diphenylphosphino)-9,9-dimethylxanthene |
| Xphos | 2-Dicyclohexylphosphino-2',4',6'-triisopropylbiphenyl |

**References**

K. Feron, R. Lim, C. Sherwood, A. Keynes, A. Brichta, P.C. Dastoor, Organic Bioeletronics: Materials and Biocompatibility. Int. J. Mol. Sci. 2018, 19, 2382 (1-21).

Gawrys, et al. Kornet, David Djurado, Stephanie Pouget, Jean-Marie Verilhac,Malgorzata Zagorska and Adam Pron J. Mater. Chem., 2010, 20, 1913-1920.

Hassner, C. Stumer, Organic Synthesis Based on Name Reactions and Unnamed Reactions, Pergamon, 1994.

Krchová, T., Kotek, J., Jirák, D., Havlíčková, J., Císařová, I., Hermann, P. Dalton Trans. 2013, 42, 15735.

Lin, B. C., Cheng, C. P., Lao, Z. P. M. J. Phy. Chem. A, 2003, 107, 5241-51.

Mann, S., Eveleigh, L., Lequin, O., Ploux, O. Anal. Biochem. 2013, 432, 90.

Rohatgi-Mukherjee, K. K. Fundamentals of Photochemistry; Wiley Eastern Limited: Calcutta, 1978.

Taniguchi, M.; Lindsey, J. S. Photochem. Photobiol. 2018, 94, 290.

C. Wang, H. Dong, L. Jiang, W. Hu, Organic semiconductor crystals. Chem. Soc. Rev. 2018, 47, 422-500.

L.M. Wysockia, L.D. Lavis, L. Advances in the chemistry of small molecule fluorescent probes. Curr. Op. In Chem. Biol. 2011, 15, 752-759.

Carolina Silva Marques, Hugo Cruz, Sandra Gago, João Paulo Ramalho, Luís Branco and Anthony J. Burke.

**Claims**

1. Compounds of formula (I) and (II):

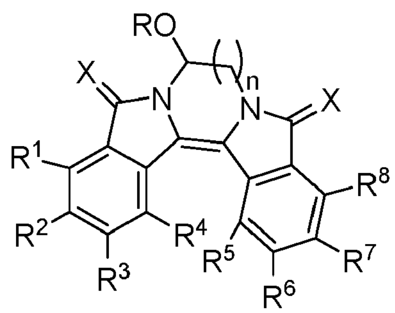

I

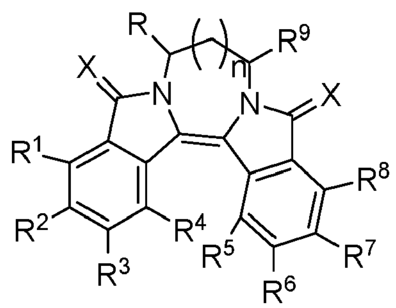

II

wherein, X is O, S or Se,

n = 1-4 for compounds of formula I and n = 0-3 for compounds of formula II,
R to $R^9$ represent: H, alkyl, aryl, vinyl, allyl, alkoxyl, OH, CN, CHO, $CO_2H$, $CO_2R$ where R represents any alkyl or aryl group, halogen: F, Br, Cl;
wherein in the case of $R^1$ to $R^8$ the adjacent pairs, including the pair R and $R^9$ optionally represent fused aromatic or cycloalkyl units.

**2.** Compounds of formula (**I**) and (**II**) according to the previous claim, wherein the compounds are chiral and exist in one enantiomeric form, either pure or enriched, as diastereomers, either enantiomerically pure or enriched, or as a racemic mixture.

**3.** Method to produce the compounds of Formula (**I**) as described in claims 1 to 2, from compounds of formula (**III**):

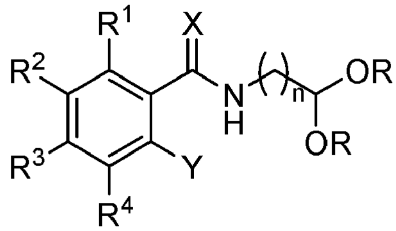

**III**

wherein Y is selected from a halogen, triflate or alkylsulfonates; $R^{1-4}$ are H, alkyl, aryl, vinyl, allyl, alkoxyl, OH, CN, CHO, $CO_2H$, $CO_2R$ where R represents any alkyl or aryl group, halogen: F, Br, Cl; and R is an alkyl or aryl group, and X is O, S or Se; and n = 1-3
**characterized by** the following steps:

- treatment of compound of formula (**III**) with iodine and acetone;
- treatment with a transition metal catalyst and a ligand, under an inert gas atmosphere in the presence of a base and a solvent;

wherein the reactions run at temperatures between 60°C and 120°C and times between 10 and 24 hours.

**4.** Method according to the previous claim, wherein the transition metal catalyst is achiral or chiral.

**5.** Method according to any of the claims 1 to 4, wherein the catalyst is selected from Pd, Rh, Ir or Cu.

**6.** Method according to any of the claims 3 to 5, wherein when the catalyst is Pd, a pre-catalyst is used between 5 and 30 mol%, selected from $PdCl_2$(dppf), $PdCl_2$ $(CH_3CN)_2$, $Pd(CH_3CN)_4(BF_4)_2$, $PdCl_2$ $(PPh_3)_2$, $Pd(OAc)_2$ and $Pd(dba)_2$.

**7.** Method according to any of the claims 3 to 6, wherein the ligands are used between 5 and 30 mol%, and are selected from $PPh_3$, (±)BINAP, $PCy_3$, P(*o*-Tol)$_3$, $PCy_3.HBF_4$, P(*t*-Bu)$_3$, Xantphos, DPEPhos, dppf, dppm, dmpe, dppp, dppb, dppe, dcpe, Buchwald-type ligands like: DavePhos, Xphos, Sphos, RuPhos, BrettPhos, (+)-BINAP, (-)-BINAP, Tol-BINAP, DIOP, DIPAMP, DioxPhos, DeguPhos, Xylyl-P-Phos, (R)-Phanephos, (*R*)-ShiP, (R)-SIPHOS, (*R, R*)-Chiraphos, $C_3$-TunePhos, TangPhos, BINAPINE, BINAPHANE, PhanePhos, Synphos, DuanPhos, Walphos and Josiphos.

**8.** Method according to any of the claims 3 to 7, wherein the base is used in a quantity between 0.5 to 3 equivalents, and is selected from triethylamine, $K_2CO_3$, $CaCO_3$, $Ba(OH)_2$, DIPEA, $K_3PO_4$, NMM, , DBU, KOH, KF and $Cs_2CO_3$.

**9.** Method according to any of the claims 3 to 8, wherein the solvent is selected from toluene, dimethyl ether, diethyl ether, dichloroethane, THF, 1,4-dioxane, acetone, acetonitrile, MeTHF, 1,2-dichloroethane, DMF, DME, DMA and NMP and used in quantities that vary from 1 to 1000 mL.

**10.** Method to produce the compounds of Formula (II) as described in claims 1 to 2, from compounds of formula (IV)

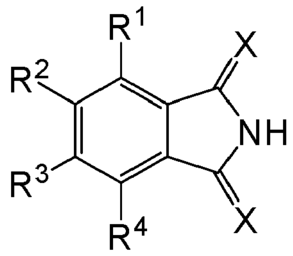

**IV**

wherein, $R^{1-4}$ are H, alkyl, aryl, vinyl, allyl, alkoxyl, OH, CN, CHO, $CO_2H$, $CO_2R$ where R represents any alkyl or aryl group, halogen: F, Br, Cl, and X is O, S or Se; **characterized by** the following steps:

- coupling of compound (**IV**) in quantities that range from 0.5 to 250 moles, with a linker in quantities that range from 1 to 3 equivalents to form a di-phthalimide intermediate in the presence of a base and a solvent;
- McMurry coupling step using $TiCl_4$ and Zn as reagents.

**11.** Method according to the previous claim, wherein the linker in compounds of formula **(II)** is obtained from: 1,2-dibromoethane, 1,2-dichloroethane, 1,2-diiodoethane, 1,3-dibromopropane, 1,3-dichloropropane, 1,3-diiodopropane, 1,4-dibromobutane, 1,4-dichlorobutane and 1,4-diiodobutane.

**12.** Method according to any of the claims 10 to 11, wherein the base is selected from NaH, NaOH, $K_2CO_3$, triethylamine, pyridine, in quantities that range from 1 to 4 molecular equivalents.

**13.** Method according to any of the claims 10 to 12, wherein the solvent is selected from toluene, DMA, DMSO, MeTHF, diethyl ether, $CH_3CN$ and used in quantities that vary from 1 to 1000 mL.

**14.** Use of compounds of formula (**I**) and (**II**), as described in claim 1 to 2, as components for optoelectronic devices.

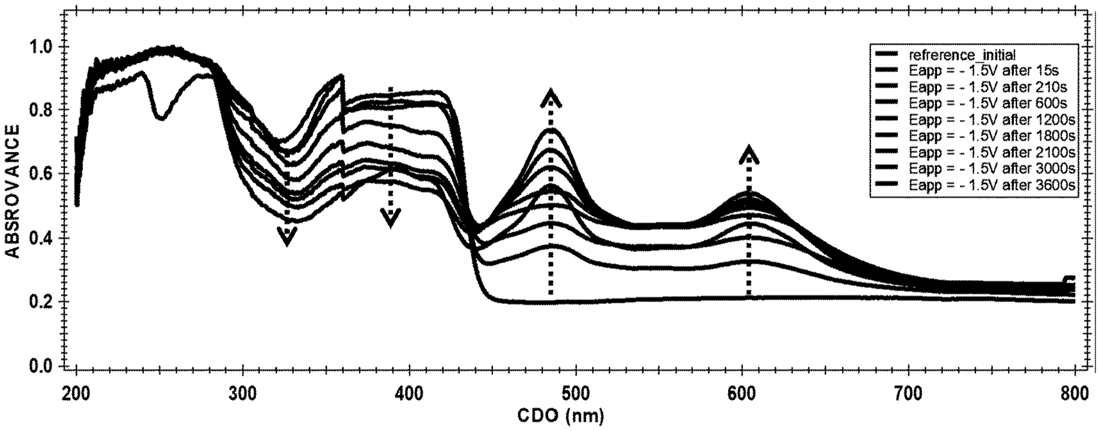


Figure 1

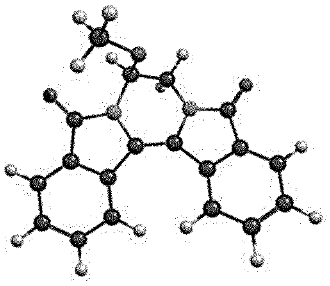

1

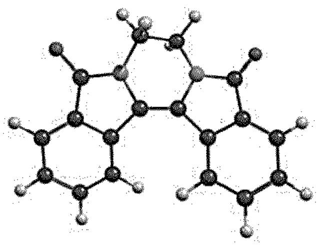

4

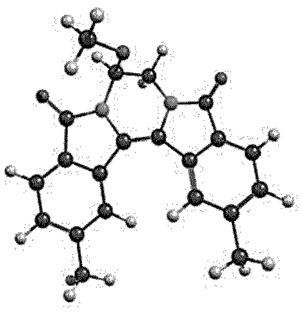

7

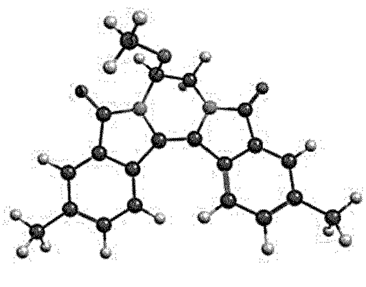

8

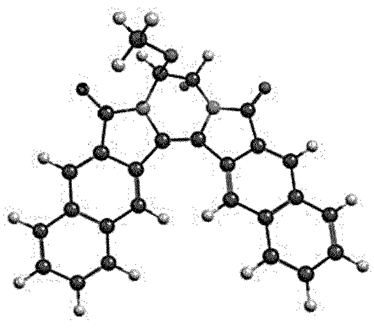

9

Figure 2

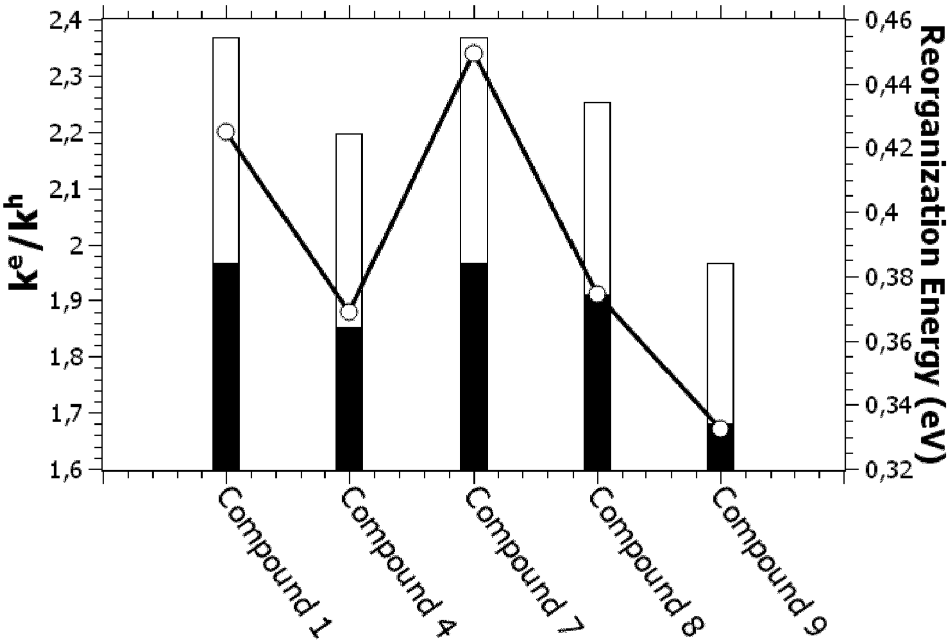

2,4
2,3
2,2
2,1
2
1,9
1,8
1,7
1,6
$k^e/k^h$
0,46
0,44
0,42
0,4
0,38
0,36
0,34
0,32
Reorganization Energy (eV)
Compound 1
Compound 4
Compound 7
Compound 8
Compound 9


Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **K. FERON ; R. LIM ; C. SHERWOOD ; A. KEYNES ; A. BRICHTA ; P.C. DASTOOR.** Organic Bioeletronics: Materials and Biocompatibility. *Int. J. Mol. Sci.,* 2018, vol. 19 (2382), 1-21
- **GAWRYS ; KORNET ; DAVID DJURADO ; STEPHANIE POUGET ; JEAN-MARIE VERILHAC ; MALGORZATA ZAGORSKA ; ADAM PRON et al.** *J. Mater. Chem.,* 2010, vol. 20, 1913-1920
- **HASSNER, C. STUMER.** Organic Synthesis Based on Name Reactions and Unnamed Reactions. Pergamon, 1994
- **KRCHOVÁ, T. ; KOTEK, J. ; JIRÁK, D. ; HAVLÍČKOVÁ, J. ; CÍSAŘOVÁ, I. ; HERMANN, P.** *Dalton Trans,* 2013, vol. 42, 15735
- **LIN, B. C. ; CHENG, C. P. ; LAO, Z. P. M.** *J. Phy. Chem. A,* 2003, vol. 107, 5241-51
- **MANN, S. ; EVELEIGH, L. ; LEQUIN, O. ; PLOUX, O.** *Anal. Biochem.,* 2013, vol. 432, 90
- **ROHATGI-MUKHERJEE, K. K.** Fundamentals of Photochemistry. Wiley Eastern Limited, 1978
- **TANIGUCHI, M. ; LINDSEY, J. S.** *Photochem. Photobiol.,* 2018, vol. 94, 290
- **C. WANG ; H. DONG ; L. JIANG ; W. HU.** Organic semiconductor crystals. *Chem. Soc. Rev.,* 2018, vol. 47, 422-500
- **L.M. WYSOCKIA ; L.D. LAVIS.** L. Advances in the chemistry of small molecule fluorescent probes. *Curr. Op. In Chem. Biol.,* 2011, vol. 15, 752-759